# EUROPEAN PATENT SPECIFICATION

(11) **EP 4 394 788 B1**
(45) Date of publication and mention of the grant of the patent: **06.05.2026**
(21) Application number: 23215506.9
(22) Date of filing: 11.12.2023
(51) Int. Cl.: G16H 20/30, A63B 24/00, A61B 5/11, G16H 50/70, G16H 50/30, G16H 50/20, A61B 5/00

(54) **PRACTICE SUPPORT APPARATUS, PRACTICE SUPPORT METHOD, AND PRACTICE SUPPORT PROGRAM**
ÜBUNGSHILFSVORRICHTUNG, ÜBUNGSHILFSVERFAHREN UND ÜBUNGSHILFSPROGRAMM
APPAREIL, PROCÉDÉ ET PROGRAMME D'AIDE À LA PRATIQUE

(30) Priority: 28.12.2022 JP 2022212395
(43) Date of publication of application: 03.07.2024
(73) Proprietor: ASICS Corporation, Kobe-shi, Hyogo 650-8555 (JP)
(72) Inventor: INABA, Ryuichiro, Kobe-shi, Hyogo, 650-8555 (JP); HIRAKAWA, Nao, Kobe-shi, Hyogo, 650-8555 (JP); KO, Yuki, Kobe-shi, Hyogo, 650-8555 (JP); NAKAYAMA, Kazunaga, Kobe-shi, Hyogo, 650-8555 (JP); NOMURA, Yasuhiro, Kobe-shi, Hyogo, 650-8555 (JP)
(74) Representative: TBK

(56) References cited:
- WO-A1-2022/110100
- WO-A1-2022/137498
- US-A1- 2013 053 990
- US-A1- 2018 369 637

## Description

### BACKGROUND OF THE INVENTION

### 1. Field of the Invention

This invention relates to a practice support system, practice support method, and practice support program. The present invention especially relates to a practice support system, practice support method, and practice support program that utilize a sensor worn by a subject to be supported.

### 2. Description of Related Art

Running ability is considered very important not only for track and field athletes but also for athletes of ball games and other sports. It is considered important to record and analyze the posture of the athletes during the movements such as walking and running.

The gait analysis system disclosed in WO2019/082376 A1 s capable of computing gait-related evaluation reference such as a gait age, an appearance age, and a beauty posture evaluation value by sequentially measuring the three-dimensional coordinates of a plurality of predetermined body feature points of a subject as the subject walks.

According to WO2019/082376 A1, a subject is capable of recognizing the objective evaluation of her/his gait by recognizing the evaluation reference computed by the gait analysis system disclosed in WO2019/082376 A1.

The gait analysis system disclosed in WO2019/082376 A1 uses a three dimensional measurement device to measure the three dimensional coordinates of a plurality of physical feature points of the subject. For example, one of the commercially available devices, Kinect (registered trademark) manufactured by Microsoft Corporation of the United States, can be used as this three dimensional measurement device.

The gait analysis system disclosed in WO2019/082376 A1 is also applicable to the analysis of the posture of the athletes during the movements. The posture of the athletes during the movements may be measured by the three dimensional measurement device to record and analyze the posture of the athletes during the movements.

Athletes complain of the needs not only to obtain an objective evaluation of their own posture while moving such as when they are walking or running but also of the need for practice menu information to improve their running ability tailored to each individual athlete, and with respect to such needs of the athletes, the gait analysis system disclosed in WO2019/082376 A1fails to consider even suggesting the practice menu information and is unable to meet such needs.

Furthermore, US 2013/053990 A1 discloses a system for analyzing an activity session performed by a user to identify one or more types of activity performed by the user during the activity session. Thereby, received activity data are processed against the classifications to and identify one or more activities performed during the activity session, wherein the multiple types of parameters defining an activity comprise any combination of two or more of: resistance experienced or generated by the user, effort exerted by the user or health of the user during the one or more activities.

Still further, US 2018/369637 A1 refers to a training system including a garment having a sensor control module connected to multiple sensor nodes via electrically-conductive fabric running along parts portions of the garment.

### SUMMARY OF THE INVENTION

It is an object of this disclosure to provide a practice support system, a practice support method, and a practice support program that are on the basis of an objective evaluation of posture of an athlete during movement such as the posture at a time of walking and running, and are capable of suggesting practice menu information suitable for each individual athlete for the purpose of improving running ability.

According to the invention, this object is achieved by a practice support apparatus according to claim 1, a practice support method according to claim 3, and a practice support program according to claim 4.

Further features and advantageous modifications are shown in the dependent claims.

That is, a practice support apparatus according to a first aspect comprises: an acquisition unit that acquires log data and evaluation data indicating an evaluation of the log data regarding exercise of a user from a sensor worn by the user; a record controller that records the log data and the evaluation data over time for each user; a model creation unit that creates model information on an exercise pattern for each user on the basis of the log data and the evaluation data; and a suggestion unit that suggests practice menu information for the user on the basis of the created model information. The model creation unit is further configured to use the range of variation in the log data for the user (1) over a certain period of time to create a relational equation for form characteristics unique to the user, and the practice menu information is suggested further based on the relational equation.

According to the invention, the suggestion unit is configured to suggest an amount of practice for each practice event of the user on the basis of the model information.

According to the invention, the model information created by the model creation unit includes risk information for quantified injury, the suggestion unit may correct posture information of the user on the basis of the risk information contained in the model information and suggest a posture on the basis of the corrected posture information, and wherein a regression model created by a regression analysis for the log data acquired in advance using an indicator of susceptibility to injury as the objective variable and indicators directed to pace, time, running time, pitch, stride, stride to height ratio, backward tilt of trunk, vertical motion, vertical motion to height ratio, body drop, pelvic drop, pelvic lift, pelvic rotation, pelvic rotation time point, horizontal impact force, kicking phase duration, ground contact time, ground contact time rate, landing impact, kicking acceleration, amount of braking, and stiffness as independent variables is used as the risk information for quantified injury.

A further aspect is that in the practice support apparatus according to the invention, the model creation unit may create the risk information for quantified injury on the basis of subjective evaluation data indicating fatigue, poor condition, and pain entered by the user.

The practice support apparatus according to the present disclosure, for example, comprises: an acquisition unit that acquires log data and evaluation data indicating an evaluation of the log data regarding exercise of a user from a sensor worn by the user; a record controller that records the log data and the evaluation data over time for each user; a model creation unit that creates model information on an exercise pattern for each user on the basis of the log data and the evaluation data; and a suggestion unit that suggests practice menu information for the user on the basis of the created model information. This allows the practice support apparatus, for example, to suggest practice menu information suitable for each individual athlete on the basis of an objective evaluation of the posture of the athlete during the movement such as when walking or running with the aim of improving the running ability.

### BRIEF DESCRIPTION OF THE DRAWINGS

Features, advantages, and technical and industrial significance of exemplary embodiments of the disclosure will be described below with reference to the accompanying drawings, in which like numerals denote like elements, and wherein:
FIG 1 is a diagram for illustrating an overview of a practice support apparatus according to an embodiment,
FIG 2 is a diagram for illustrating an example of use of a wearable sensor according to the present embodiment,
FIGS. 3A and 3B are diagrams for illustrating an example of wearing of the wearable sensor according to the present embodiment,
FIG 4 is a diagram for illustrating an overview of functions of the practice support apparatus according to the present embodiment,
FIG 5 is a diagram for illustrating an example of a configuration of the wearable sensor according to the present embodiment,
FIG 6 is a diagram for illustrating an example of a configuration of the practice support apparatus according to the present embodiment,
FIG. 7 is a block diagram for illustrating an example of a functional configuration of the practice support apparatus according to the present embodiment,
FIG. 8A is a flowchart of a practice support program according to the present embodiment, and
FIG. 8B is a flowchart of a practice support program according to another embodiment.

### DETAILED DESCRIPTION OF EMBODIMENTS

### Overview of Practice Support Apparatus 30

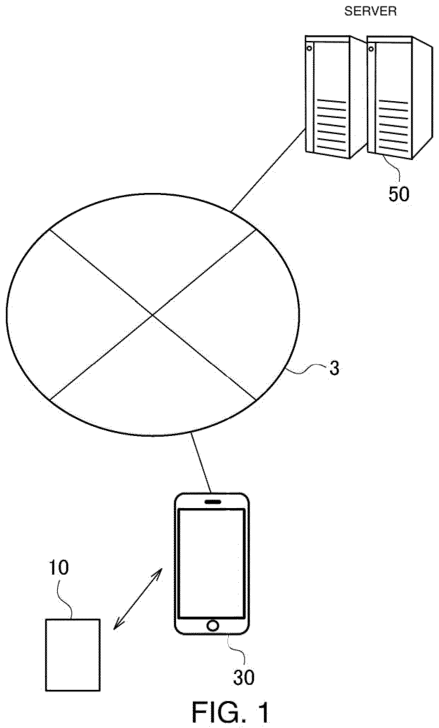

With reference to FIG. 1, an overview of the practice support apparatus 30 according to an embodiment will be described.

The practice support apparatus 30 corresponds to an information processing apparatus represented by a personal computer (hereinafter, referred to as "PC"), notebook PC, tablet PC, and smartphone, for example, in which the practice support program described below is installed.

The practice support apparatus 30 is wired to or wirelessly connected to a network 3 including the Internet, and transmits and receives various data to and from the server 50 described below.

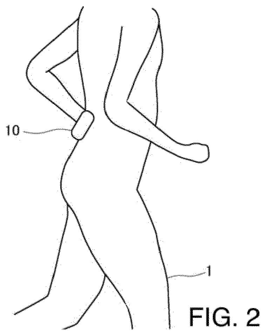

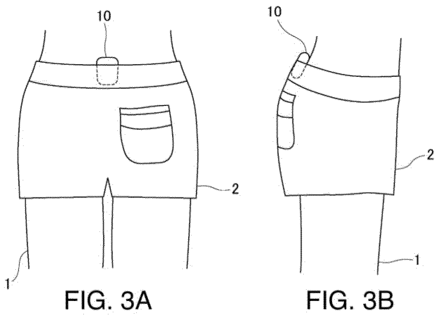

The practice support apparatus 30 transmits and receives data to and from a sensor (hereinafter, referred to as the wearable sensor 10) worn by a user 1 as a subject of the practice support apparatus 30 (see FIGS. 2, 3A, and 3B).

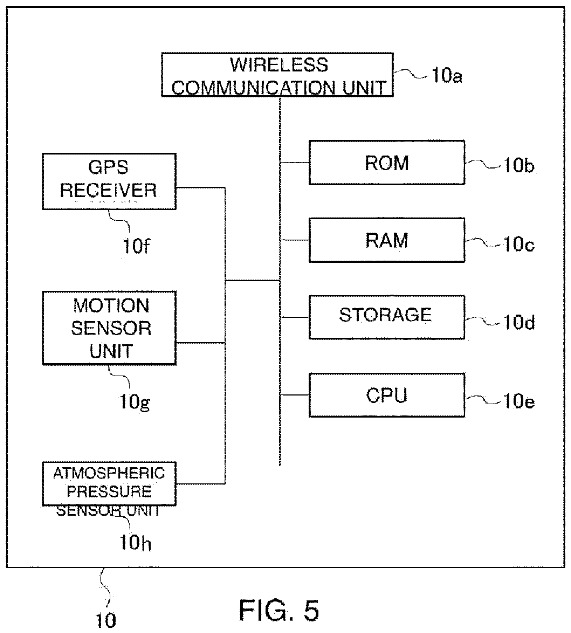

The wearable sensor 10 includes a motion sensor unit 10g, an atmospheric pressure sensor unit 10h, and a receiver 10f of the global positioning system (hereinafter, referred to as GPS) as described below (see FIG. 5).

The motion sensor unit 10g includes a triaxial acceleration sensor, triaxial gyro sensor (angular velocity sensor), and triaxial geomagnetic sensor.

The triaxial acceleration sensor detects the acceleration generated in the wearable sensor 10 by decomposing it into vector components in the mutually orthogonal x, y, and z axis directions, and measures the direction and magnitude of motion (change in velocity), vibration, and impact, for example.

The triaxial gyro sensor (angular velocity sensor) captures the rotation and orientation changes that occur in the wearable sensor 10 as an angular velocity, which is decomposed into vector components in the mutually perpendicular x, y, and z axis directions.

The triaxial geomagnetic sensor measures the geomagnetic field received by the wearable sensor 10 for each vector component in the perpendicular x, y, and z axis directions to measure the direction and magnitude of the geomagnetic field and detect changes in the direction of the wearable sensor 10.

The atmospheric pressure sensor unit 10h corresponds to a sensor that measures atmospheric pressure.

The GPS receiver 10f corresponds to a functional unit that includes an antenna and other components for receiving signals from several satellites in the sky, and the position of the wearable sensor 10 is determined on the basis of the received signals. The GPS is directed to a satellite positioning system of the United States, but in addition to the GPS, the receiver 10f also supports signals from MICHIBIKI, a Japanese satellite positioning system, and GLONASS, a satellite positioning system of the Russian Federation.

The wearable sensor 10 transmits various data acquired by the motion sensor unit 10g, the atmospheric pressure sensor unit 10h, and the GPS receiver 10f to the practice support apparatus 30.

On the basis of the various data acquired from the wearable sensor 10, the practice support apparatus 30 acquires at least 22 indicators related to the running form of the user during walking or running on the movement as log data. The practice support apparatus 30 associates and records time information indicating the date and time in which the log data has been acquired and position information acquired from the GPS receiver 10f indicating the location where the log data has been acquired with each of the acquired log data.

The log data corresponds to historical information on the training performed by the user. Specifically, the information may include the 22 indicators listed below as well as the position information, time information, distance information, and velocity information acquired by the wearable sensor 10.

The 22 indicators that serve as log data are directed to pace, time, running time, pitch, stride, stride to height ratio, backward tilt of trunk, vertical motion, vertical motion to height ratio, body drop, pelvic drop, pelvic lift, pelvic rotation, pelvic rotation time point, horizontal impact force, kicking phase duration, ground contact time, ground contact time rate, landing impact, kicking acceleration, amount of braking, and stiffness, each of which will be described below.

The 22 indicators may be broadly classified into basic indicators, indicators related to the trunk, indicators related to the waist movements, and indicators related to the leg movements.

In addition to the 22 indicators, the log data includes, as indicators, the position information, time information, distance information, and velocity information on the basis of the data acquired by the GPS receiver 10f, motion sensor unit 10g, and atmospheric pressure sensor unit 10h of the wearable sensor 10.

The position information corresponds to information such as that related to the position measured on the basis of the signals received by the GPS receiver 10f. The time information is on the basis of the time information contained in the signals received by the GPS receiver 10f. The distance information corresponds to the difference between the position information at a certain point in time and the position information after a predetermined time has elapsed. The velocity information may be acquired by computation on the basis of the distance information and the time information.

The basic indicators correspond to indicators as reference used for evaluating running, and may include "pace," "time," "running time," "pitch," "stride," and "stride to height ratio."

The indicator "pace" corresponds to the time it takes to run one kilometer, computed on the basis of the lap time, which is substantially equal to the time when the lap distance is measured for one kilometer.

The "time" corresponds to the time required to pass the lap distance.

The "running time" corresponds to the amount of time that the user has been determined to have run excluding walking and pauses.

The "pitch (number of foot rotations per minute)" [steps/min] corresponds to the number of steps per minute. At the same pace, a greater numerical value shows that the user is running at a smaller rhythm. In each pace, a greater value shows that the user is running at a rhythm smaller than the average.

The "stride" [m] corresponds to a distance traveled in one step from one ground contact to the next. A greater numerical value of the stride shows a greater distance traveled per step.

The "stride to height ratio" corresponds to the ratio of stride to height. Often a height ratio is used since stride is greatly influenced by the leg length. The greater the value of the stride to height ratio in the reference value for each pace is, the greater the score for the "strength of movement" becomes.

The indicators related to the trunk correspond to indicators of the movement of the trunk during running, such as "backward tilt of the trunk."

The "backward tilt of trunk" [°] corresponds to a movement in which the pelvis and trunk are tilted backward at the time of grounding. The greater a numerical value of the backward tilt of trunk is, the more unstable the posture of the trunk becomes. Independent from the running pace, the less a numerical value of the backward tilt of the trunk is, the greater the score for the "stable posture" becomes.

The indicators related to the waist movements are used to evaluate the waist movement during running, for example, and may correspond to "vertical motion," "vertical motion to height ratio," "body drop," "pelvic drop," "pelvic lift," "pelvic rotation," "pelvic rotation time point," "horizontal impact force," and "kicking phase duration."

The "vertical motion" [cm] is directed to the vertical motion of the body during the running. The greater a numerical value of the vertical motion value is, the greater the sinking and bouncing of the run becomes.

The "vertical motion to height ratio" [%] corresponds to a numerical value indicating the vertical motion to the height ratio. The smaller a value of the vertical motion to height ratio in the reference value for each pace is, the greater the score for the "less load ground contact" becomes.

The "body drop" [%] corresponds to a movement in which the knees bend and the position of the waist drops after the ground contact. The body drop is expressed by a percentage of height (100%), the greater the numerical value is, the deeper the sink is. The smaller the value of the body drop in the reference value for each pace is, the greater the score for the "linked movement of the whole body with the pelvis as the axis" becomes.

The "pelvic drop" [°] corresponds to a movement in which the pelvis on the side opposite the grounded leg tilts downward. The greater the pelvic drop is, the more unstable the posture of the pelvis during the grounding is. The smaller the value of the pelvic drop in the reference value for each pace is, the greater the score for the "stable posture" becomes.

The "pelvic lift" [°] corresponds to the movement to pull back the pelvis that has dropped to the swing leg side from the ground contact to kick-out. Negative values indicate kicking out with the pelvis on the swing leg side in a lower position, and the greater positive values indicate kicking out with the pelvis on the swing leg side raised higher.

The "pelvic rotation" [°] corresponds to a movement that rotates the pelvis. The greater a numerical value of the pelvic rotation is, the more powerful the run with a greater pelvic rotation is. The greater the value of the pelvic rotation in the reference value for each pace is, the greater the score for the "strength of movement" becomes.

The "pelvic rotation time point" corresponds to the gap between the moment of the ground contact and the time point of the pelvic rotation. Zero shows that the time point of the ground contact is consistent with the time point of the pelvic rotation. The greater the negative value is, the more the run emphasizes a movement in which the pelvis begins to rotate earlier than the ground contact and the foot is drawn directly under the body. In contrast, the greater the positive value is, the more the run emphasizes a movement in which the swing leg side is swung forward with a greater pelvic rotation after the ground contact. Independent from the running pace, the greater the value of the pelvic rotation time point is, the greater the score for the "linked movement of the whole body with the pelvis as the axis" becomes.

The "horizontal impact force" [m/s2] corresponds to the magnitude of the impact applied to the left and right sides of the body. In general, the greater a numerical value of the horizontal impact force is, the greater the instability of the run in the lateral direction is.

The "kicking phase duration" [ms] corresponds to the time between when the body drops after the ground contact and when the foot leaves the ground. The greater a numerical value of the kicking phase duration is, the longer time the run takes to kick each step. The smaller the value of the kicking phase duration in the reference value for each pace is, the greater the score for the "linked movement of the whole body with the pelvis as the axis" becomes.

The indicators related to the leg movements are used to evaluate the leg movements during running, for example, and may correspond to the "ground contact time," "ground contact time rate," "landing impact," "kicking acceleration," "amount of braking," and "stiffness."

The "ground contact time" [ms] corresponds to the time from the ground contact until the foot leaves the ground after kicking out. In general, the faster the pace is, the shorter the ground contact time becomes.

The "ground contact time rate" [%] corresponds to a numerical value indicating the ground contact time as a percentage of one cycle (time from the ground contact to the next).

The "landing impact" [m/s2] corresponds to the magnitude of the impact on the body immediately after the ground contact. In general, the greater a numerical value of the landing impact is, the greater the load on the legs becomes. The smaller the value of the landing impact in the reference value for each pace is, the greater the score for the "less load ground contact" becomes.

The "kicking acceleration" [m/s2] corresponds to the magnitude of the acceleration to kick out the ground. In general, the greater a numerical value of the kicking acceleration is, the stronger the force with which the ground is kicked becomes. The smaller the value of the kicking acceleration in the reference value for each pace is, the greater the score for the "less load ground contact" becomes.

The "amount of braking" [m/s] corresponds to the amount of braking after the ground contact. In general, the greater a numerical value of the amount of braking is, the greater the braking per step in the run is.

The "stiffness" [kN/m-kg] corresponds to the stiffness of a spring when the entire leg is considered as the "spring." In general, the greater a numerical value of the stiffness is, the less the sinking after the ground contact is, and the running becomes the "harder spring" in which the runner kicks out the ground in a shorter ground contact time. Independent of the pace, the smaller the left-right difference is, the greater the score for the "symmetry" is, in which the left or right leg with the greater value approaches the "leading" side.

These 22 indicators are aggregated into six indicators that serve as evaluation data. The evaluation data is computed in consideration of the velocity information with respect to these 22 indicators, and is further expressed as a 10-grade evaluation value by comparing the data with the previously acquired data of others. The evaluation data is presented to the user as a hexagonal radar chart with these six indicators as apices.

The six indicators are directed to: linked movement of the whole body with the pelvis as the axis, strength of movement, smooth center of gravity shift, less load ground contact, stable posture, and symmetry described below.

The "linked movement of the whole body with the pelvis as the axis" is directed to a score that indicates whether the athlete is able to move to use the portions of the body in a well-balanced manner, and is computed on the basis of the body drop, pelvic rotation time point, and kicking phase duration from among the 22 indicators listed above.

The "strength of movement" is directed to a score indicating whether the athlete is able to perform the movement for extending the stride for a powerful run, and is computed on the basis of the stride to height ratio, pelvic lift, and pelvic rotation from among the 22 indicators listed above.

The "smooth center of gravity shift" is directed to a score indicating whether left-right shaking and braking are kept to a minimum to move forward efficiently, and is computed on the basis of the horizontal impact force and amount of braking from among the 22 indicators listed above.

The "less load ground contact" is directed to a score indicating whether the athlete is able to keep the force on the waist small enough to minimize the load caused by the impact, and is computed on the basis of the vertical motion to height ratio, landing impact, and kicking acceleration from among the 22 indicators listed above.

The "stable posture" is directed to a score indicating whether the unstable pelvic movement fails to occur to reduce the load on the muscles and joints, and is computed on the basis of the backward tilt of trunk and the pelvic drop among the 22 indicators listed above.

The "symmetry" is directed to a score indicating whether the lateral sides of the body are maintained even, i.e., with no difference in features during the running, and is computed on the basis of the scores for the six indicators listed above.

### Regarding Wearing of Wearable Sensor 10 by User 1

Next, with reference to FIGS. 2, 3A and 3B, the wearing of the wearable sensor 10 by the user 1 will be described. FIG. 2 illustrates an example of the use of a wearable sensor according to the present embodiment, and FIGS. 3A and 3B illustrate an example of the wearing of the wearable sensor according to the present embodiment.

As shown in FIG. 2, the wearable sensor 10 is mounted on the central portion behind the waist of the user 1 (a portion equally distanced from both sides). As shown in FIGS. 3A and 3B, the wearable sensor 10 may be mounted on a predetermined position on the waist of the user 1 by fastening it to the waist elastic of pants 2 with a clip (not shown).

The wearable sensor 10 worn behind the waist of the user 1 detects the acceleration, angular velocity, and geomagnetic field acting behind the waist of the user 1, and also measures the position of the user 1. Further, since the signals received by the GPS receiver 10f include the current time, data related to the acceleration, angular velocity, geomagnetic field, and positioning detected by the wearable sensor 10 can be recorded along with the detected time.

On the basis of the data related to the acceleration, angular velocity, and geomagnetic field acting behind the waist of the user 1, the acceleration, and angular velocity, for example, generated in the portions of the body including the head, arms, and legs of the user 1 are estimated to compute the 22 indicators (pace, time, running time, pitch, stride, stride to height ratio, backward tilt of trunk, vertical motion, vertical motion to height ratio, body drop, pelvic drop, pelvic lift, pelvic rotation, pelvic rotation time point, horizontal impact force, kicking phase duration, ground contact time, ground contact time rate, landing impact, kicking acceleration, amount of braking, and stiffness) listed above. One method of computing these 22 indicators is to create a regression model on the basis of a regression analysis in which the data detected by the wearable sensor 10 is used as the explanatory variables and the values of each of the above 22 indicators are used as the explained variables.

### Regarding Overview of Practice Support Apparatus 30

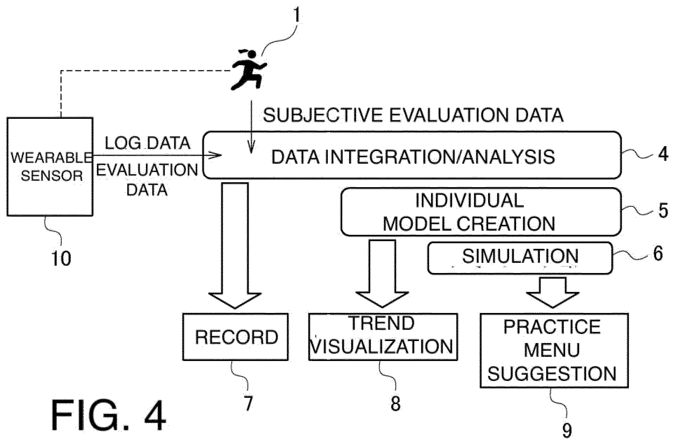

With reference to FIG. 4, an overview of the functions of the practice support apparatus 30 will be described. FIG. 4 is a diagram for illustrating an overview of the functions of the practice support apparatus 30 according to the present embodiment.

The practice support apparatus 30 acquires the log data and evaluation data described above from the wearable sensor 10.

The evaluation data refers to information on the evaluation of the log data compared with the data of others acquired in advance. Specifically, when the log data is acquired from the wearable sensor 10, the evaluation data is represented by an evaluation value (1, 2, 3, ..., 10) on the basis of 10-grade evaluation for each of the six indicators listed above, which is computed on the basis of the 22 indicators as log data. The evaluation data is automatically added to the log data by the wearable sensor 10 or the practice support apparatus 30, and is associated and recorded with the log data. The data of others acquired in advance may correspond to, for example, the average value of other users of the same age as that of the user, the average value of other users with the same experience or ability as that of the user, or the average value of other users in the same sport as that of the user, and may be directed to a value to be set in advance. Further, the data of others acquired in advance may be directed to a record of a famous athlete or an athlete that the user 1 likes, and in this case, the evaluation data is assigned to the log data as evaluation data with an evaluation value from a 10-grade evaluation on the basis of a comparison with this athlete.

The practice support apparatus 30 acquires the subjective evaluation of the acquired log data by the user 1 as subjective evaluation data. The practice support apparatus 30 associates these acquired data with each other, performs "data integration and analysis" 4 and "records" 7 them. Further, on the basis of these data, "individual model creation" 5 for the user 1 and the "trend visualization" 8 are performed. Moreover, the "simulation" 6 is performed by analyzing newly acquired log data on the basis of the created individual model, and the "suggestion of the practice menu information" 9 is implemented for the user 1.

The practice menu information corresponds to a combination of multiple practice events that the user 1 performs to improve his/her ability in a certain exercise pattern, and specifically, it is directed to a predetermined schedule of practice progression in a week.

### Regarding Configuration of Wearable Sensor 10

With reference to FIG. 5, a configuration of the wearable sensor 10 will be described. FIG. 5 is a diagram for illustrating an example of a configuration of the wearable sensor 10 according to the present embodiment.

The wearable sensor 10 includes functional units such as a wireless communication unit 10a, a read only memory (ROM) 10b, a random access memory (RAM) 10c, a storage 10d, a central processing unit (CPU) 10e, a GPS receiver 10f, a motion sensor unit 10g, and an atmospheric pressure sensor unit 10h.

The wireless communication unit 10a corresponds to a functional unit for transmitting and receiving data to and from the practice support apparatus 30, and is used for data communication via near field communication such as Bluetooth (registered trademark) or wired connection.

The ROM 10b and storage 10c can be used as storage devices and store the firmware and data used by the firmware that are needed for the operation of the wearable sensor 10.

The wearable sensor 10 stores the firmware in the ROM 10b or storage 10c and writes the firmware in the main memory configured by the RAM 10c, for example. The CPU 10e then accesses the main memory in which the firmware has been written, executes the firmware, and controls the functional units.

The storage 10c also functions as a buffer (buffer memory) to temporarily store data related to the signals detected by the GPS receiver 10f, motion sensor unit 10g, and atmospheric pressure sensor unit 10h until they are transmitted toward the practice support apparatus 30.

The wearable sensor 10 has an outline of about 40 mm (width) x 62 mm (height) x 18 mm (depth) and a mass of about 37 g. It is designed to be lightweight and compact, and does not get in the way while the user 1 is training while wearing the wearable sensor 10. The power source for the wearable sensor 10 is a lithium-ion rechargeable battery with a continuous operating time of approximately 20 hours.

### Regarding Configuration of Practice Support Apparatus 30

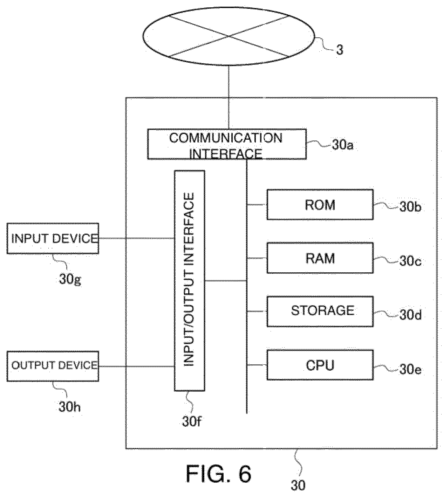

With reference to FIG. 6, the configuration of the practice support apparatus 30 will be described. FIG. 6 illustrates an example of the configuration of the practice support apparatus 30 according to the present embodiment.

The practice support apparatus 30 has functional units such as communication interface 30a, ROM 30b, RAM 30c, storage 30d, CPU 30e, and input/output interface 30f. The practice support apparatus 30 also includes an input device 30g and an output device 30h as its external devices.

The communication interface 30a mainly serves to input and output data to and from the network 3.

The storage 30d may be used as a storage device and store the practice support program, various applications, and various data used by the applications, for example, described below, which are needed for the operation of the practice support apparatus 30.

The input/output interface 30f transmits and receives data, for example, to and from the input device 30g and output device 30h of the practice support apparatus 30. The input device 30g may include a keyboard, mouse, and scanner (not shown), for example. The output device 30h may include a monitor, printer, and speakers (not shown), for example, i.e., peripheral devices.

The practice support apparatus 30 stores the practice support program described below in the ROM 30b or storage 30d, and reads the practice support program in the main memory configured by the RAM 30c, for example. The CPU 30e accesses the main memory that contains the practice support program, executes the practice support program, and controls the functional units.

### Regarding Functional Configuration of Practice Support Apparatus 30

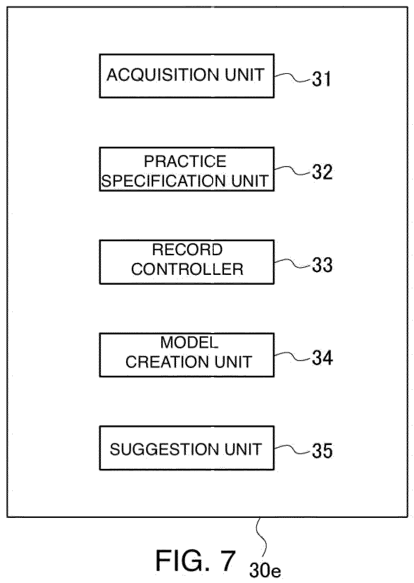

Next, with reference to FIG. 7, an example of the functional configuration of the practice support apparatus 30 will be described. FIG. 7 is a block diagram for illustrating an example of a functional configuration of the practice support apparatus 30 according to the present embodiment.

By executing the practice support program, the practice support apparatus 30 allows the CPU 30e to serve as the acquisition unit 31, practice specification unit 32, record controller 33, model creation unit 34, and suggestion unit 35 as functional units.

The acquisition unit acquires the log data and evaluation data indicating an evaluation of the log data regarding the exercise of the user 1 from the sensor (wearable sensor) 10 worn by the user 1.

The practice specification unit 32 classifies the log data acquired by the acquisition unit 31 by time for each practice event and specifies a time point of implementation of each practice event on the basis of the association between the practice event of the practice performed by the user 1 and the position information, time information, distance information, and velocity information contained in the log data.

The practice event is directed to a type of practice (training) to improve the ability, skill, or performance of an exercise pattern as described below. The exercise pattern refers to various sports events, specifically track and road events. For example, the track events may include short, middle, and long distance races, hurdle races, steeplechase races, and the field events may include leaps such as high jump and long jump, and throws such as the shot put and discus throw. The road events may include marathons, and walking races, for example.

The following is a specific description of the practice events using a middle distance race as an example of the exercise pattern. The practice events for the middle distance races may include, for example, starting dash, dash, tempo run, jog, wave run, mark run, set run, and overspeed run. These features specific to the practice events are extracted from the position, time, distance, and speed information contained in the log data acquired by the wearable sensor 10 in advance.

The practice specification unit 32 classifies the log data acquired by the acquisition unit 31 for each practice event by time and specifies the time point of each practice event on the basis of the association between with the log data and the previously extracted features.

The record controller 33 records the log data and evaluation data over time for each user 1.

The record controller 33 records the log data and evaluation data in the storage 30d of the practice support apparatus 30.

The record controller 33 associates and records the practice events specified by the practice specification unit 32 with the time point of the implementation of the practice events.

This allows the practice support apparatus 30 to reproduce and record the practice menu information that has been performed by the user 1.

The model creation unit 34 specifies possible fluctuation ranges of the log data and creates model information on the association between the fluctuation ranges.

The log data is acquired from multiple practices and stored by the record controller 33.

The model creation unit 34 may also specify the variation ranges of the evaluation data acquired from the multiple practices and stored by the record controller 33, and create the association between the variation ranges of the evaluation data as model information.

The model information uses the range of variation in the log data for the user 1 over a certain period of time to create a relational equation for the form characteristics unique to the user 1, such as "the increase in the landing impact is steeper when the stride exceeds 1.00." This allows the suggestion unit 35, which will be described below, to make suggestions on the basis of the individual models to the user 1 in consideration of the characteristics of the movements that vary from the user 1 to another user 1.

The model information may correspond to a multiple regression equation for multiple regression analysis, where each of the variation ranges of the indicators contained in the log data acquired by the acquisition unit 31 during a certain period of time for the user 1 is assigned to an explanatory variable and the landing impact is the explained variable. The landing impact is one of the indicators showing the susceptibility to injury. The standard partial regression coefficient of this multiple regression analysis corresponds to an indicator indicating the importance of each explanatory variable in the multiple regression equation computed by the multiple regression analysis, and represents the importance of the variation range of each indicator contained in the log data assigned to each explanatory variable. That is, the greater the absolute value of the standard partial regression coefficient is, the more important the variation range of the indicator is for the landing impact. Accordingly, the magnitude of the importance of the variation ranges of the log data with each other for the landing impact may be expressed by the magnitude of the standard partial regression coefficient.

If the evaluation data is directed to, for example, an evaluation value of 10-grade evaluation (1, 2, 3, ..., 10), the model information is assigned the evaluation value corresponding to the position within the variation range to become a model as a reference of evaluation for the newly acquired log data.

The movement information is directed to information on the body movements associated with the training of the user 1. Specifically, the information includes, from among the 22 indicators listed above, pitch, stride, stride to height ratio, vertical motion, vertical motion to height ratio, pelvic lift, pelvic rotation, pelvic rotation time point, kicking phase duration, ground contact time, ground contact time rate, landing impact, kicking acceleration, amount of braking, and stiffness.

The posture information is directed to information on the body posture associated with the training of the user 1, specifically, the backward tilt of trunk, the body drop, the pelvic drop, and the horizontal impact force.

The model creation unit 34 creates quantified injury risk information on the basis of the subjective evaluation data indicating fatigue, poor condition, and pain entered by the user 1.

The injury risk information is directed to information that represents the susceptibility of the user 1 to the injury, specifically the regression model described below.

The subjective evaluation data is directed to information on the evaluation on the basis of the subjectivity of the user 1, which is entered by the user 1 to the practice support apparatus 30 for the log data, and is directed to an evaluation of the fatigue, poor condition, and pain that the user 1 has felt about the training, expressed, for example, as one of very bad, bad, normal, good, or very good, which is recorded along with the log data.

Further, the subjective evaluation data may be associated and entered with the underperforming area as a factor in the evaluation. The underperforming area may be directed to, for example, the back, right and left hip joints, right and left thighs, right and left knees, right and left calves, right and left shins, right and left Achilles' tendons, right and left ankles, right and left heels, or right and left toes.

The suggestion unit 35 suggests practice menu information for the user 1 on the basis of the created model information.

The practice menu information is prepared in advance, which is associated with the amount of difference between the model information as target (hereinafter, referred to as target model information) and the model information created by the model creation unit 34.

The suggestion unit 35 suggests practice menu information for the user 1 on the basis of the amount of difference between the model information created by the model creation unit 34 and the target model information.

Alternatively, the suggestion unit 35 may suggest practice menu information for the user 1 on the basis of the model information created by the model creation unit 34, using a learning model that has machine-learned the association between the target model information and the practice menu information in advance.

The target model information is directed to, for example, the model information that is the average for the same gender and age as those of the user 1.

The suggestion unit 35 is also capable of making suggestions in accordance with the goals of the user 1. For example, for the user 1 that plans to compete in a full marathon in six months, training menu information is suggested for each of the following periods: preparation period, speed request period, sufficiently running period, race simulation period, and tapering (adjustment) period.

In this case, the suggestion unit 35 is capable of suggesting practice menu information adapted to each period by using learning models adapted to each period.

The suggestion unit 35 suggests the amount of practice for each practice event for the user 1 on the basis of the model information.

The model information created by the model creation unit 34 includes quantified injury risk information, and the suggestion unit 35 corrects the posture information of the user on the basis of the risk information included in the model information and suggests a posture on the basis of the corrected posture information.

The suggestion unit 35 is capable of predicting the likelihood of injury when the user 1 performs the practice with the current form characteristics on the basis of the suggested practice menu information in accordance with the goals of the user 1 and the relational expression between the log data variable ranges specific to the user 1 created by the model creation unit 34. Further, on the basis of this, the practice menu or the amount of practice may be changed to reduce the susceptibility to injury, or the improvement of the posture may be encouraged, thereby improving the relational expression between the variable ranges specific to the user 1.

For example, the user 1 with the landing impact being prone to be greater at the time when running at a pace of more than four minutes per kilometer is expected to be more prone to be susceptible to injury when performing a practice that includes more paces exceeding four minutes per kilometer. Accordingly, the suggestion unit 35 is capable of reducing the risk of injury of the user 1 by suggesting a practice menu that reduces the practice pace or promotes the postural improvement so that the landing impact fails to increase even at a pace exceeding four minutes per kilometer. Further, the suggestion unit 35 is capable of reducing the risk of injury by suggesting, to the user 1 with the landing impact being prone to be greater when running a distance exceeding 10 kilometers, a practice menu that reduces the practice distance, or by suggesting a practice menu that encourages strength training so that the landing impact fails to increase even if the distance exceeds 10 kilometers.

The regression model created by the regression analysis for the log data acquired in advance using the indicator of the susceptibility to injury as the objective variable and the 22 indicators mentioned above as independent variables is used as the injury risk information.

A single indicator such as landing impact may be used as the indicator indicating the susceptibility to injury as the explained variable, or the explained variable may be represented by a composite combination of indicators. For example, a value computed by multiplication of indicators over a certain period of time, such as cumulative landing impact computed by adding up the number of steps taken in a month to the landing impact, may be used as an indicator indicating susceptibility to injury, which is the explained variable. An indicator related to the variation of the indicator over a period of time such as the variation in cumulative landing impact over a month may also be used as the explained variable. The explanatory variable for determining the explained variable may correspond to the variation range of the indicators contained in the log data acquired by the acquisition unit 31. When the variable predicting the susceptibility to injury is used as the explained variable, not only the variation range of the indicator included in the log data acquired by the acquisition unit 31 but also the distance and velocity information acquired by adding up or averaging the indicators contained in the log data over a certain period, such as the accumulated travel distance and average velocity, may be used as the explanatory variables.

### Regarding Practice Support Method and Practice Support Program

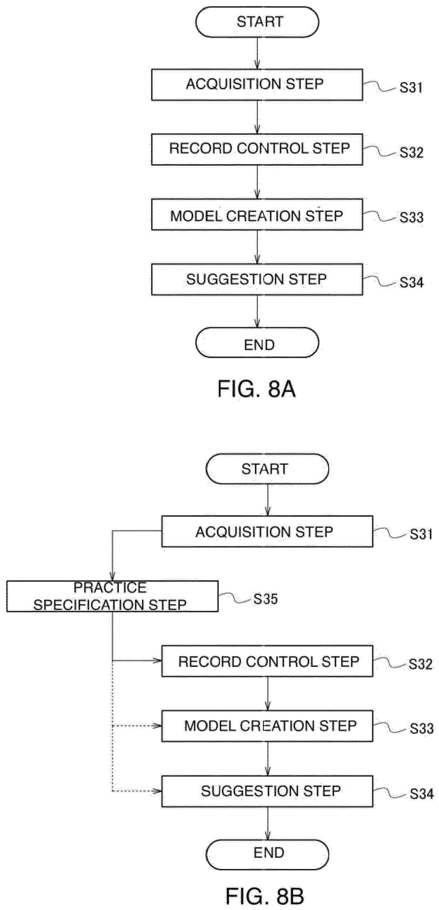

Next, with reference to FIGS. 8A and 8B, a practice support method according to the present embodiment will be described along with the practice support program. FIGS. 8A and 8B are flowcharts each illustrating an example of a practice support program.

As shown in FIG. 8A, the practice support program includes an acquisition step S31, a record control step S32, a model creation step S33, and a suggestion step S34.

The practice support apparatus 30 reads the practice support program stored in the ROM 30b or storage 30d into the main memory configured by the RAM 30c, and the CPU 30e executes the practice support program by accessing the main memory.

The practice support program causes the CPU30eof the practice support apparatus 30 to embody the functions such as the acquisition function, the record control function, the model creation function, and the suggestion function.

Although the case in which these functions are processed in the order shown in the flowchart in FIG. 8A is illustrated as an example, this case is not limited thereto, and the practice support program may be executed in which the order of these functions is changed as appropriate.

Since the description of the functions above are redundant with the description above of the acquisition unit 31, record controller 33, model creation unit 34, and suggestion unit 35 of the practice support apparatus 30, the detailed description thereof is omitted.

The acquisition function acquires the log data and evaluation data indicating the evaluation of the log data regarding the exercise of the user 1 from the sensor (wearable sensor) 10 worn by the user 1 (S31: acquisition step).

The record control function records the log data and evaluation data over time for each user 1 (S32: record control step).

The model creation function creates the model information on the exercise pattern for each user 1 on the basis of the log data and evaluation data (S33: model creation step).

The suggestion function suggests practice menu information for the user 1 on the basis of the created model information (S34: suggestion step).

The practice support program may also include a practice specification step S35 (see FIG. 8B) between the acquisition step S31 and the record control step S32. In this case, the practice support program further includes a practice specification function for the CPU 30e of the practice support apparatus 30.

The practice specification function classifies the log data acquired by the acquisition function by time for each practice event and specifies a time point of implementation of each practice event on the basis of the association between practice event of the practice performed by the user 1 and position information, time information, distance information, and velocity information contained in the log data.

The practice specification step S35 may be provided between the acquisition step S31 and the model creation step S33, or between the acquisition step S31 and the suggestion step S34 (see FIG 8B).

According to the embodiment described above, the practice support apparatus 30 is capable of acquiring the log data of the training of the user 1 by allowing the user 1 to wear the wearable sensor 10, which does not interfere with the training, and also recording the log data by specifying the time of implementation for each practice event. Accordingly, the practice support apparatus 30 is capable of recording the log data acquired while reproducing the practice menu information performed by the user 1.

Further, according to the embodiment described above, the practice support apparatus 30 is capable of suggesting the practice menu information on the basis of the log data acquired by the wearable sensor 10. The practice menu information corresponds to data that describes the contents of the practice.

In the above embodiment, the practice support apparatus 30 may display the monthly cumulative practice time, the overall cumulative practice time, and the practice time for each practice event on the output device 30h. Further, the practice event and training history information determined by the practice support apparatus 30 may be transmitted via the communication interface 30a through the network 3 to another information terminal on the network 3. In this information terminal, the type of the determined practice event and training history information may be displayed. Further, the practice support apparatus 30 may display multiple types of characters to the output device 30h on the basis of the training history information, and may perform a variable performance. Moreover, in the practice support apparatus 30, the suggestion unit 35 is capable of changing the practice menu information for training by weighting the model information in accordance with the emotion on the basis of the keywords related to the emotion entered through the input device 30g.

### [Description of the Reference Numerals]

1: user
2: pants
3: network
4: data integration and analysis
5: individual model creation
6: simulation
7: record
8: trend visualization
9: suggestion of practice menu information
10: wearable sensor
10a: wireless communication unit
10b: read only memory (ROM)
10c: random access memory (RAM)
10d: storage
10e: central processing unit (CPU)
10f: GPS receiver
10g: motion sensor unit
10h: atmospheric pressure sensor unit
30: practice support apparatus
30a: communication interface
30b: read only memory (ROM)
30c: random access memory (RAM)
30d: storage
30e: central processing unit (CPU)
30f: input/output interface
30g: input device
30h: output device
31: acquisition unit
32: practice specification unit
33: record controller
34: model creation unit
35: suggestion unit
50: server

A practice support apparatus (30) comprises: an acquisition unit (31) that acquires log data and evaluation data indicating an evaluation of the log data regarding exercise of a user (1) from a sensor (10) worn by the user (1); a record controller (33) that records the log data and the evaluation data over time for each user (1); a model creation unit (34) that creates model information on an exercise pattern for each user (1) on the basis of the log data and the evaluation data; and a suggestion unit (35) that suggests practice menu information for the user (1) on the basis of the created model information.

## Claims

1. A practice support apparatus (30) comprising:
an acquisition unit (31) configured to acquire log data and evaluation data indicating an evaluation of the log data regarding exercise of a user (1) from a sensor (10) worn by the user (1);
a record controller (33) configured to record the log data and the evaluation data over time for the user (1);
a model creation unit (34) configured to create model information on an exercise pattern for the user (1) on the basis of the log data and the evaluation data; and
a suggestion unit (35) configured to suggest practice menu information for the user (1) on the basis of the created model information,
wherein
the model creation unit (34) is further configured to use a range of variation in the log data for the user (1) over a certain period of time to create a relational equation for form characteristics unique to the user (1), and
the practice menu information is suggested further based on the relational equation,
wherein the suggestion unit (35) is configured to suggest an amount of practice for the practice event of the user (1) on the basis of the model information, and
wherein
the model information created by the model creation unit (34) includes risk information for quantified injury,
the suggestion unit (35) is configured to correct posture information of the user (1) on the basis of the risk information contained in the model information and suggest a posture on the basis of the corrected posture information, and
**characterized in that**
a regression model created by a regression analysis for the log data acquired in advance using an indicator of susceptibility to injury as the objective variable and indicators directed to pace, time, running time, pitch, stride, stride to height ratio, backward tilt of trunk, vertical motion, vertical motion to height ratio, body drop, pelvic drop, pelvic lift, pelvic rotation, pelvic rotation time point, horizontal impact force, kicking phase duration, ground contact time, ground contact time rate, landing impact, kicking acceleration, amount of braking, and stiffness as independent variables is used as the risk information for quantified injury.

2. The practice support apparatus (30) according to claim 1, wherein the model creation unit (34) is configured to create the risk information for quantified injury on the basis of subjective evaluation data indicating fatigue, poor condition, and pain entered by the user (1).

3. A practice support method causing a computer to execute the steps of:
acquiring log data and evaluation data related to exercise of a user (1) from a sensor (10) worn by the user (1);
recording the log data and the evaluation data over time for the user (1);
creating model information on an exercise pattern for the user (1) on the basis of the log data and the evaluation data; and
suggesting practice menu information for the user (1) on the basis of the created model information,
using a range of variation in the log data for the user (1) over a certain period of time to create a relational equation for form characteristics unique to the user (1), and
suggesting the practice menu information further based on the relational equation,
wherein the suggesting includes suggesting an amount of practice for the practice event of the user (1) on the basis of the model information, and
wherein
the created model information includes risk information for quantified injury,
the suggesting further includes correcting posture information of the user (1) on the basis of the risk information contained in the model information and suggesting a posture on the basis of the corrected posture information, and **characterized in that**
a regression model created by a regression analysis for the log data acquired in advance using an indicator of susceptibility to injury as the objective variable and indicators directed to pace, time, running time, pitch, stride, stride to height ratio, backward tilt of trunk, vertical motion, vertical motion to height ratio, body drop, pelvic drop, pelvic lift, pelvic rotation, pelvic rotation time point, horizontal impact force, kicking phase duration, ground contact time, ground contact time rate, landing impact, kicking acceleration, amount of braking, and stiffness as independent variables is used as the risk information for quantified injury.

4. A practice support program causing a computer to embody the functions of:
acquiring log data and evaluation data related to exercise of a user (1) from a sensor (10) worn by the user (1);
recording the log data and the evaluation data over time for a user (1);
creating model information on an exercise pattern for the user (1) on the basis of the log data and the evaluation data; and
suggesting practice menu information for the user (1) on the basis of the created model information,
using a range of variation in the log data for the user (1) over a certain period of time to create a relational equation for form characteristics unique to the user (1), and
suggesting the practice menu information further based on the relational equation,
wherein the suggesting includes suggesting an amount of practice for the practice event of the user (1) on the basis of the model information, and
wherein
the created model information includes risk information for quantified injury,
the suggesting further includes correcting posture information of the user (1) on the basis of the risk information contained in the model information and suggesting a posture on the basis of the corrected posture information, and
**characterized in that**
a regression model created by a regression analysis for the log data acquired in advance using an indicator of susceptibility to injury as the objective variable and indicators directed to pace, time, running time, pitch, stride, stride to height ratio, backward tilt of trunk, vertical motion, vertical motion to height ratio, body drop, pelvic drop, pelvic lift, pelvic rotation, pelvic rotation time point, horizontal impact force, kicking phase duration, ground contact time, ground contact time rate, landing impact, kicking acceleration, amount of braking, and stiffness as independent variables is used as the risk information for quantified injury.

## Patentansprüche

1. Trainingsunterstützungsvorrichtung (30), mit:
einer Bezugseinheit (31), die konfiguriert ist, um von einem vom Benutzer (1) getragenen Sensor (10) Protokolldaten und Auswertungsdaten zu beziehen, die eine Auswertung der Protokolldaten bezüglich der körperlichen Betätigung des Benutzers (1) angeben;
einer Aufzeichnungssteuerung (33), die konfiguriert ist, um die Protokolldaten und die Auswertungsdaten für den Benutzer (1) über die Zeit aufzuzeichnen;
einer Modellerstellungseinheit (34), die konfiguriert ist, um auf der Grundlage der Protokolldaten und der Auswertungsdaten Modellinformationen zu einem Trainingsmuster für den Benutzer (1) zu erstellen; und
einer Vorschlageinheit (35), die konfiguriert ist, um auf der Grundlage der erstellten Modellinformationen Trainingsmenüinformationen für den Benutzer (1) vorzuschlagen,
wobei
die Modellerzeugungseinheit (34) weiterhin konfiguriert ist, einen Variationsbereich in den Protokolldaten für den Benutzer (1) über einen bestimmten Zeitraum zu verwenden, um eine relationale Gleichung für Formmerkmale zu erstellen, die für den Benutzer (1) einzigartig sind, und
die Trainingsmenüinformationen weiterhin auf der Grundlage der relationalen Gleichung vorgeschlagen werden,
wobei die Vorschlageinheit (35) konfiguriert ist, um auf der Grundlage der Modellinformationen einen Trainingsumfang für die Trainingsaktivität des Benutzers (1) vorzuschlagen, und
wobei
die von der Modellerstellungseinheit (34) erstellten Modellinformationen Risikoinformationen für quantifizierte Verletzungen enthalten,
die Vorschlageinheit (35) konfiguriert ist, um Haltungsinformationen des Benutzers (1) auf der Grundlage der in den Modellinformationen enthaltenen Risikoinformationen zu korrigieren und auf der Grundlage der korrigierten Haltungsinformationen eine Haltung vorzuschlagen, und
**dadurch gekennzeichnet, dass**
ein Regressionsmodell, das durch eine Regressionsanalyse der zuvor erfassten Protokolldaten unter Verwendung eines Indikators für die Verletzungsanfälligkeit als Zielvariable und Indikatoren für Tempo, Zeit, Laufzeit, Schrittlänge, Schritt-zu-Körpergrößen-Verhältnis, Rückwärtsneigung des Rumpfes, vertikale Bewegung, Verhältnis von vertikaler Bewegung zu Körpergröße, Körperabsenkung, Beckenabsenkung, Beckenanhebung, Beckenrotation, Zeitpunkt der Beckenrotation, horizontale Aufprallkraft, Dauer der Abstoßphase, Bodenkontaktzeit, Bodenkontaktzeitrate, Aufprallkraft bei der Landung, Beschleunigung beim Abstoßen, Bremswirkung und Steifigkeit als unabhängige Variablen erstellt wurde, als Risikoinformationen für quantifizierte Verletzungen verwendet wird.

2. Trainingsunterstützungsvorrichtung (30) gemäß Anspruch 1, wobei die Modellerstellungseinheit (34) konfiguriert ist, um die Risikoinformationen für quantifizierte Verletzungen auf der Grundlage von subjektiven Bewertungsdaten zu erstellen, die Ermüdung, schlechte Verfassung und Schmerzen anzeigen und vom Benutzer (1) eingegeben wurden.

3. Trainingsunterstützungsverfahren, das einen Computer veranlasst, die Schritte ausführen:
Beziehen von Protokolldaten und Bewertungsdaten bezüglich des Trainings eines Benutzers (1) von einem vom Benutzer (1) getragenen Sensor (10);
Aufzeichnen der Protokolldaten und der Bewertungsdaten über die Zeit für den Benutzer (1);
Erstellen von Modellinformationen zu einem Trainingsmuster für den Benutzer (1) auf der Grundlage der Protokolldaten und der Bewertungsdaten; und
Vorschlagen von Trainingsmenüinformationen für den Benutzer (1) auf der Grundlage der erstellten Modellinformationen,
Verwenden eines Variationsbereichs in den Protokolldaten für den Benutzer (1) über einen bestimmten Zeitraum, um eine relationale Gleichung für die für den Benutzer (1) spezifischen Formmerkmale zu erstellen, und
Vorschlagen der Trainingsmenüinformationen weiterhin auf der Grundlage der relationalen Gleichung,
wobei das Vorschlagen das Vorschlagen eines Trainingsumfangs für die Trainingseinheit des Benutzers (1) auf der Grundlage der Modellinformationen umfasst, und
wobei
die erstellten Modellinformationen Risikoinformationen für quantifizierte Verletzungen umfassen,
das Vorschlagen weiterhin das Korrigieren von Haltungsinformationen des Benutzers (1) auf der Grundlage der in den Modellinformationen enthaltenen Risikoinformationen und das Vorschlagen einer Haltung auf der Grundlage der korrigierten Haltungsinformationen umfasst, und
**dadurch gekennzeichnet, dass**
ein Regressionsmodell, das durch eine Regressionsanalyse der zuvor erfassten Protokolldaten unter Verwendung eines Indikators für die Verletzungsanfälligkeit als Zielvariable und von Indikatoren für Tempo, Zeit, Laufzeit, Schrittlänge, Schritt-zu-Körpergrößen-Verhältnis, Rückwärtsneigung des Rumpfes, vertikale Bewegung, Verhältnis von vertikaler Bewegung zu Körpergröße, Körperabsenkung, Beckenabsenkung, Beckenanhebung, Beckenrotation, Zeitpunkt der Beckenrotation, horizontale Aufprallkraft, Dauer der Abstoßphase, Bodenkontaktzeit, Bodenkontaktzeitrate, Aufprallkraft bei der Landung, Beschleunigung beim Abstoßen, Bremswirkung und Steifigkeit als unabhängige Variablen erstellt wurde, als Risikoinformationen für quantifizierte Verletzungen verwendet wird.

4. Trainingsunterstützungsprogramm, das einen Computer veranlasst, die folgenden Funktionen auszuführen:
Beziehen von Protokolldaten und Auswertungsdaten in Bezug auf das Training eines Benutzers (1) von einem Sensor (10), der vom Benutzer (1) getragen wird;
Aufzeichnen der Protokolldaten und der Auswertungsdaten über die Zeit für einen Benutzer (1);
Erstellen von Modellinformationen zu einem Trainingsmuster für den Benutzer (1) auf der Grundlage der Protokolldaten und der Auswertungsdaten; und
Vorschlagen von Trainingsmenüinformationen für den Benutzer (1) auf der Grundlage der erstellten Modellinformationen,
Verwenden eines Variationsbereichs in den Protokolldaten für den Benutzer (1) über einen bestimmten Zeitraum, um eine relationale Gleichung für die für den Benutzer (1) spezifischen Formmerkmale zu erstellen, und
Vorschlagen der Trainingsmenüinformationen weiterhin auf der Grundlage der relationalen Gleichung,
wobei das Vorschlagen das Vorschlagen eines Trainingsumfangs für die Trainingseinheit des Benutzers (1) auf der Grundlage der Modellinformationen umfasst, und
wobei
die erstellten Modellinformationen Risikoinformationen für quantifizierte Verletzungen umfassen,
das Vorschlagen weiterhin das Korrigieren von Haltungsinformationen des Benutzers (1) auf der Grundlage der in den Modellinformationen enthaltenen Risikoinformationen und das Vorschlagen einer Haltung auf der Grundlage der korrigierten Haltungsinformationen umfasst, und
**dadurch gekennzeichnet, dass**
ein Regressionsmodell, das durch eine Regressionsanalyse der zuvor erfassten Protokolldaten unter Verwendung eines Indikators für die Verletzungsanfälligkeit als Zielvariable und von Indikatoren für Tempo, Zeit, Laufzeit, Schrittlänge, Schritt-zu-Körpergrößen-Verhältnis, Rückwärtsneigung des Rumpfes, vertikale Bewegung, Verhältnis von vertikaler Bewegung zu Körpergröße, Körperabsenkung, Beckenabsenkung, Beckenanhebung, Beckenrotation, Zeitpunkt der Beckenrotation, horizontale Aufprallkraft, Dauer der Abstoßphase, Bodenkontaktzeit, Bodenkontaktzeitrate, Aufprallkraft bei der Landung, Beschleunigung beim Abstoßen, Bremswirkung und Steifigkeit als unabhängige Variablen erstellt wurde, als Risikoinformation für quantifizierte Verletzungen verwendet wird.

## Revendications

1. Appareil d'aide à la pratique (30) comprenant :
une unité d'acquisition (31) configurée pour acquérir des données de journal et des données d'évaluation indiquant une évaluation des données de journal concernant l'exercice d'un utilisateur (1) à partir d'un capteur (10) porté par l'utilisateur (1) ;
un contrôleur d'enregistrement (33) configuré pour enregistrer les données de journal et les données d'évaluation dans le temps pour l'utilisateur (1) ;
une unité de création de modèle (34) configurée pour créer des informations de modèle sur un modèle d'exercice pour l'utilisateur (1) sur la base des données de journal et des données d'évaluation ; et
une unité de suggestion (35) configurée pour suggérer à l'utilisateur (1) des informations de menu de pratique sur la base des informations de modèle créées,
dans lequel
l'unité de création de modèle (34) est en outre configurée pour utiliser une plage de variation dans les données de journal pour l'utilisateur (1) sur une certaine période de temps afin de créer une équation relationnelle pour des caractéristiques de forme propres à l'utilisateur (1), et
les informations de menu de pratique sont suggérées en outre sur la base de l'équation relationnelle,
dans lequel l'unité de suggestion (35) est configurée pour suggérer une quantité de pratique pour l'événement de pratique de l'utilisateur (1) sur la base des informations de modèle, et
dans lequel
les informations de modèle créées par l'unité de création de modèle (34) comprennent des informations de risque pour une blessure quantifiée,
l'unité de suggestion (35) est configurée pour corriger les informations de posture de l'utilisateur (1) sur la base des informations de risque contenues dans les informations de modèle et suggérer une posture sur la base des informations de posture corrigées, et
**caractérisé en ce que**
un modèle de régression créé par une analyse de régression pour les données de journal acquises à l'avance en utilisant un indicateur de susceptibilité aux blessures comme variable objective et des indicateurs orientés vers le rythme, le temps, le temps de course, le tangage, la foulée, le rapport foulée/hauteur, l'inclinaison vers l'arrière du tronc, le mouvement vertical, le rapport mouvement vertical/hauteur, la chute corporelle, la chute pelvienne, le soulèvement pelvien, la rotation pelvienne, le point temporel de rotation pelvienne, la force d'impact horizontale, la durée de la phase de coup de pied, le temps de contact avec le sol, la vitesse du temps de contact avec le sol, l'impact à l'atterrissage, l'accélération du coup de pied, la quantité de freinage et la rigidité comme variables indépendantes sont utilisées comme informations de risque pour les blessures quantifiées.

2. Appareil d'aide à la pratique (30) selon la revendication 1, dans lequel l'unité de création de modèle (34) est configurée pour créer les informations de risque pour une blessure quantifiée sur la base de données d'évaluation subjectives indiquant la fatigue, un mauvais état de santé et une douleur entrées par l'utilisateur (1).

3. Procédé d'aide à la pratique permettant à un ordinateur d'exécuter les étapes suivantes :
l'acquisition de données de journal et de données d'évaluation relatives à l'exercice d'un utilisateur (1) à partir d'un capteur (10) porté par l'utilisateur (1) ;
l'enregistrement des données de journal et des données d'évaluation dans le temps pour l'utilisateur (1) ;
la création d'informations de modèle sur un modèle d'exercice pour l'utilisateur (1) sur la base des données de journal et des données d'évaluation ; et
la suggestion à l'utilisateur (1) des informations de menu de pratique sur la base des informations de modèle créées,
l'utilisation d'une plage de variation dans les données de journal pour l'utilisateur (1) sur une certaine période de temps pour créer une équation relationnelle pour des caractéristiques de forme propres à l'utilisateur (1), et
la suggestion des informations de menu de pratique basées en outre sur l'équation relationnelle, dans lequel la suggestion consiste à suggérer une quantité de pratique pour l'événement de pratique de l'utilisateur (1) sur la base des informations de modèle, et
dans lequel
les informations de modèle créées comprennent des informations de risque pour les blessures quantifiées,
la suggestion consiste en outre à corriger les informations de posture de l'utilisateur (1) sur la base des informations de risque contenues dans les informations de modèle et à suggérer une posture sur la base des informations de posture corrigées, et **caractérisé en ce que**
un modèle de régression créé par une analyse de régression pour les données de journal acquises à l'avance en utilisant un indicateur de susceptibilité aux blessures comme variable objective et des indicateurs orientés vers le rythme, le temps, le temps de course, le tangage, la foulée, le rapport foulée/hauteur, l'inclinaison vers l'arrière du tronc, le mouvement vertical, le rapport mouvement vertical/hauteur, la chute corporelle, la chute pelvienne, le soulèvement pelvien, la rotation pelvienne, le point temporel de rotation pelvienne, la force d'impact horizontale, la durée de la phase de coup de pied, le temps de contact avec le sol, la vitesse du temps de contact avec le sol, l'impact à l'atterrissage, l'accélération du coup de pied, la quantité de freinage et la rigidité comme variables indépendantes sont utilisées comme informations de risque pour les blessures quantifiées.

4. Programme d'aide à la pratique permettant à un ordinateur de comprendre les fonctions suivantes :
l'acquisition de données de journal et de données d'évaluation relatives à l'exercice d'un utilisateur (1) à partir d'un capteur (10) porté par l'utilisateur (1) ;
l'enregistrement des données de journal et des données d'évaluation dans le temps pour un utilisateur (1) ;
la création d'informations de modèle sur un modèle d'exercice pour l'utilisateur (1) sur la base des données de journal et des données d'évaluation ; et
la suggestion à l'utilisateur (1) des informations de menu de pratique sur la base des informations de modèle créées,
l'utilisation d'une plage de variation dans les données de journal pour l'utilisateur (1) sur une certaine période de temps pour créer une équation relationnelle pour des caractéristiques de forme propres à l'utilisateur (1), et
la suggestion des informations de menu de pratique basées en outre sur l'équation relationnelle, dans lequel la suggestion consiste à suggérer une quantité de pratique pour l'événement de pratique de l'utilisateur (1) sur la base des informations de modèle, et
dans lequel
les informations de modèle créées comprennent des informations de risque pour les blessures quantifiées,
la suggestion consiste également à corriger les informations de posture de l'utilisateur (1) sur la base des informations de risque contenues dans les informations de modèle et à suggérer une posture sur la base des informations de posture corrigées, et
**caractérisé en ce que**
un modèle de régression créé par une analyse de régression pour les données de journal acquises à l'avance en utilisant un indicateur de susceptibilité aux blessures comme variable objective et des indicateurs orientés vers le rythme, le temps, le temps de course, le tangage, la foulée, le rapport foulée/hauteur, l'inclinaison vers l'arrière du tronc, le mouvement vertical, le rapport mouvement vertical/hauteur, la chute corporelle, la chute pelvienne, le soulèvement pelvien, la rotation pelvienne, le point temporel de rotation pelvienne, la force d'impact horizontale, la durée de la phase de coup de pied, le temps de contact avec le sol, la vitesse du temps de contact avec le sol, l'impact à l'atterrissage, l'accélération du coup de pied, la quantité de freinage et la rigidité comme variables indépendantes sont utilisées comme informations de risque pour les blessures quantifiées.
